# EUROPÄISCHE PATENTANMELDUNG

(11) **EP 2 907 565 A1**
(43) Veröffentlichungstag der Anmeldung: **19.08.2015**
(21) Anmeldenummer: 14155338.8
(22) Anmeldetag: 17.02.2014
(51) Int. Cl.: B01D 61/24, B01D 61/28, B01D 61/32, B01D 61/58

(54) **Dialyse-Einheit zum kontinuierlichen Puffer- bzw. Medienaustausch aus einer Proteinlösung**

(71) Anmelder: Bayer Technology Services GmbH, 51368 Leverkusen (DE)
(72) Erfinder: Schwan, Peter, 51373 Leverkusen (DE); Lenz, Lutz-Peter, 42655 Solingen (DE); Baumarth, Kerstin, 42329 Wuppertal (DE); Lobedann, Martin, 51069 Köln (DE)
(74) Vertreter: BIP Patents

(57) **Zusammenfassung**

Die Erfindung betrifft eine Ultrafiltrationsunit zum kontinuierlichen Puffer- bzw. Medienaustausch, ein Verfahren zum kontinuierlichen Puffer- bzw. Medienaustausch in der Ultrafiltrationsunit, sowie eine Anlage insbesondere zur (semi-)kontinuierlichen Produktion von biopharmazeutischen und biologischen makromolekulare Produkten insbesondere wie Proteinen, z. B. monoklonale Antikörper und Impfstoffe umfassend die erfindungsgemäße Ultrafiltrationsunit.

## Beschreibung

Die Erfindung betrifft eine Ultrafiltrationsunit zum kontinuierlichen Puffer- bzw. Medienaustausch, ein Verfahren zum kontinuierlichen Puffer- bzw. Medienaustausch in der Ultrafiltrationsunit, sowie eine Anlage insbesondere zur (semi-)kontinuierlichen Produktion von biopharmazeutischen und biologischen makromolekulare Produkten insbesondere wie Proteinen, z. B. monoklonale Antikörper und Impfstoffe umfassend die erfindungsgemäße Ultrafiltrationsunit.

Ein kontinuierlicher Betrieb bedeutet im Sinne der Anmeldung, dass die Zufuhr von Feedstrom in den Bioreaktor und die Abfuhr des Produktstroms aus der Produktionsanlage pausenlos erfolgen, wobei manche Schritte semi-kontinuierlich sein können.

Die stark regulierte pharmazeutische Produktion erfordert einen großen zeitlichen, technischen und personellen Aufwand für die Bereitstellung gereinigter und sterilisierter Bioreaktoren und Gewährleistung eines keimfreien Produkts. Um Kreuz-kontaminationen bei einem Produktwechsel in einer Multi-Purpose-Anlage oder zwischen zwei Produktchargen sicher zu vermeiden, wird außer der Reinigung eine sehr aufwändige Reinigungsvalidierung benötigt, welche bei einer Prozessadaption ggf. wiederholt werden muss.

Dies gilt sowohl für das Upstream-Processing USP, d. h. die Herstellung biologischer Produkte in Fermentern als auch für das Downstream-Processing DSP, d. h. die Aufreinigung der Fermentationsprodukte.

Gerade bei der Fermentation ist eine keimfreie Umgebung für eine erfolgreiche Kultivierung essentiell. Zur Sterilisation von Batch- oder Fedbatch-Fermentern kommt in der Regel die SIP-Technik zum Einsatz (SIP = sterilizable-in-place).

Der durch die Bereitstellungsprozeduren bedingte Nutzungsausfall der Reaktoren kann insbesondere bei kurzen Nutzungsperioden und häufigem Produktwechsel in der Größenordnung der Reaktorverfügbarkeit liegen. Betroffen sind im USP der biotechnologischen Produktion z. B. die Prozessschritte der Medienherstellung und Fermentation und im DSP das Solubilisieren, Einfrieren, Auftauen, pH-Adjustieren, Produkttrennung wie z. B. durch Chromatographie, Fällen, oder Kristallisieren, das Umpuffem und die Virusinaktivierung.

Um der Forderung an ein schnelles und flexibles Neubeschicken der Produktionsanlage unter Wahrung maximaler Sauberkeit und Keimfreiheit gerecht zu werden, erfreuen sich auf dem Markt Konzepte für eine kontinuierliche Produktion bevorzugt mit Einwegtechnologie eines ständig wachsenden Interesses.

WO2012/078677 beschreibt ein Verfahren und eine Anlage zur kontinuierlichen Aufbereitung von biopharmazeutischen Produkten durch Chromatographie und deren Integration in einer Produktionsanlage insbesondere in einer Einweg-Anlage. Obwohl WO2012/078677 Ansätze zur kontinuierlichen Produktion von biopharmazeutischen und biologischen Produkten liefert, reicht die offenbarte Lösung in der Praxis nicht aus.

Ein Verfahren zur Herstellung von biopharmazeutischen und biologischen Produkten umfasst üblicherweise folgende Produktionsschritte, die miteinander verschaltet werden:
1. Perfusionskultur
2. Zellrückhaltesystem,
   alternativ zu Schritt 1 und 2 ist eine Fedbatchkultur.
3. Zellabtrennung
4. Puffer- bzw. Medienaustausch bevorzugt mit Konzentrierung
5. BioBurden Reduzierung bevorzugt durch Sterilfiltration
6. Capture Chromatographie

Üblicherweise werden weitere Schritte zur weiteren Reinigung des Produktstroms durchgeführt, insbesondere:
7. Virus Inaktivierung
8. Neutralisation
9. Optional eine weitere BioBurden Reduzierung (Sterilfiltration)

Angesichts der hohen Qualitätsstandards bei der Herstellung von Biopharmazeutika werden üblicherweise darüber hinaus folgen folgende Schritte:
10. Chromatographische Zwischen- und Feinreinigung
11. BioBurden Reduzierung z. B. Sterilfiltration
12. Virenfiltration
13. Pufferaustausch und bevorzugt Konzentrierung
14. Sterilfiltration.

Bei der obig beschrieben Herstellung stellen Zellen in einem Fermenter mit Nährstofflösung ein biologisches Produkt her. Die Nährstofflösung ist dabei auch ein ideales Wachstumsmedium für Bakterien und Sporen.

Eine Aufgabe ist es daher das Verkeimungsrisiko in den nachfolgenden Prozessschritten zu verringern. Die Lösung dieser Aufgabe ist umso dringlicher je länger ein Prozess dauert, insbesondere in einem kontinuierlichen Prozess. Bei der Herstellung von Proteinen wird mindestens an einem Prozessschritt ein Puffer- bzw. Medienaustausch (=Umpufferung) der Produktlösung durchgeführt.

Weiterhin sind mehr oder wenige aufwändige Einstellungen eines oder mehrerer Parameter des Feedstroms/Produktstroms, insbesondere Konzentration, Volumen/Flussrate, pH und Leitfähigkeit für manche Prozessschritte erforderlich. Insbesondere können verschiedene chromatographische Schritte verschiedene Neueinstellungen eines oder mehrerer Parameter des Feedstroms erfordern. Für einen kontinuierlichen Prozess muss vorzugsweise zudem eine kontinuierliche Verfahrenslösung gefunden werden.

WO2012/078677 geht auf ein Puffer- bzw. Medienaustausch oder auf die Einstellung des Feedstroms insbesondere im Vorfeld der Chromatographie Unit nicht ein, so dass die Anwendbarkeit der offenbarten kontinuierlichen Anlage sich auf Chromatographietypen begrenzt, die keine besondere Einstellungen insbesondere bezüglich Konzentration, pH und Leitfähigkeit erfordern.

Als Maßnahme zur Einstellung der Parameter des Feeds in einem Batch-Verfahren ist die Umpufferung im Stand der Technik bekannt. Üblicherweise wird eine batchweise Diafiltration mit einer Ultrafiltrationsmembran verwendet. Dabei wird eine Produktlösung in einen Behälter vorgelegt. Die Lösung wird über eine Ultrafiltrationsmembran im Kreis gepumpt, wobei das Produkt möglichst von der Membran zurückgehalten wird, während Salze/Puffer durch die Membran als Permeat abgetrennt werden. Der Füllstand im Produktbehälter wird gewöhnlich konstant gehalten, in dem die Permeatmenge durch Waschflüssigkeit ersetzt wird. Die Restkonzentration der Salze im Produkt errechnet sich dann idealerweise wie folgt: C=Co*(-Waschmenge/Menge Produktlösung), wobei Co die Anfangskonzentration des Salzes ist. Dieser Prozess wird in der Literatur meist fälschlicherweise als kontinuierliche Diafiltration benannt, obwohl es ein Batch Prozess ist, da kein Feedstrom in den Behälter kontinuierlich zugeführt wird und aus dem Behälter kein Produktstrom kontinuierlich abgeführt wird. Dies ist dem Umstand geschuldet, dass kontinuierliche Puffer dem System zugeführt werden bis der Batch fertig prozessiert ist [Alois Jungbauer, "Continuous downstream processing of biopharmaceuticals", Trends in Biotech., 2013 (8), 479-492, WO2009017491].

Ultrafiltrationsmodule werden auch in der Hämodialyse, Hämofiltration und Hämodiafiltration eingesetzt. Die Hämodialyse unterscheidet sich von den anderen Ultrafiltrationsverfahren dadurch, dass es sich um einen rein diffusiven und nicht druckgesteuerten Prozess handelt. Verfahrenstechnisch ist jedoch auch die Hämodialyse ein absatzweiser Prozess bei dem der menschliche Körper als Produktbehälter angesehen werden kann und das Blut des Patienten in der Regel über mehrere Stunden gewaschen werden muss. Die Dialyse ist außerdem im Labor zur Entsalzung und Umpufferung von Proteinlösungen im Stand der Technik bekannt. Dabei ist die Dialyse aufgrund der vermeintlich langsamen Diffusion nur ein Verfahren für kleinere Labormengen [ThermoScientific http://www. piercenet.com/browse. cfm?fld I D=5753AFD9-5056-8A 76-4E 13-5F9E9B4324DA, CN102703550].

Die dynamische Dialyse verwendet die Flussdynamik, um sowohl die Rate als auch die Effizienz der Dialyse zu erhöhen. Durch die Zirkulation der Probe und/oder des Dialysats entsteht der höchstmögliche Konzentrationsgradient, um die Dialysezeit erheblich zu verkürzen. Andere Vorteile der Anströmung sind, dass die Membranverschmutzung verhindert wird, während in manchen Fällen ein Druckdifferential erzeugt wird. Diese zusätzliche treibende Kraft erhöht die hypoosmotische Stoffübergangsrate über die semipermeable Membran und ermöglicht die Probenkonzentration während des Dialysevorgangs. Je nach der Anwendung gibt es zwei grundlegende Verfahren, in dem das Dialysat strömt und die Probe entweder statisch oder strömend sein kann. Diese Verfahren werden aus verschiedenen Gründen und für verschiedene Zwecke in der dynamischen Dialyse verwendet. Strömt die Probe wird sie im Stand der Technik in einem Kreissystem umfassend einen Produktkessel geführt [http://de.spectrumlabs.com/dialysis/Dynamic.html].

Eine kontinuierliche Diafiltration bedeutet im Sinne der Anmeldung, dass ein Feedstrom und ein Waschstrom kontinuierlich der Ultrafiltrationseinheit zugeführt werden. Ein semi-kontinuierlicher Betrieb bedeutet im Sinne der Anmeldung, was im Allgemeinen als Ansatz oder Batch Aufreinigung verstanden wird, d. h. dass ein oder mehrere Ansätze einer Proteinlösung aus einem oder mehreren Behältern kontinuierlich der Ultrafiltrationseinheit zugeführt werden, wobei auch hier die Waschflüssigkeit kontinuierlich zugeführt wird.

Eine tatsächliche kontinuierliche Diafiltration lässt sich im Stand der Technik dadurch realisieren, dass man mehrere Diafiltrationsstufen im Gleichstrom bzw. im Gegenstrom miteinander verschaltet. Der apparative Aufwand ist hierfür jedoch entsprechend groß [Alois Jungbauer, "Continuous downstream processing of biopharmaceuticals", Trends in Biotech., 2013 (8), 479-492,].

Eine einfache kontinuierliche Lösung zur Einstellung der Feed-Parameter und insbesondere zur Umpufferung des Produktstroms ist bisher nicht bekannt.

Es bestand daher die erste Aufgabe darin, eine Lösung bereit zu stellen, die eine Einstellung des Produktstroms, insbesondere die Einstellung der Konzentration, pH und/oder Leitfähigkeit eines kontinuierlichen Produktstroms, durch Umpufferung, ermöglicht. Insbesondere soll die Lösung flexibel sein und die Einstellung des Produktstroms an die Erfordernisse eines oder mehrerer unterschiedlicher chromatographischer Reinigungsschritte ermöglichen.

Die Aufgabe wurde durch die Nutzung von mindestens einem im Gleichstrom, Gegenstrom oder Kreuzstrom, vorzugsweise im Gegenstrom betriebenen Hohlfaser-Ultrafiltrationsmodul (=Kapillar-Ultrafiltrationsmodul), bevorzugt Hämodialysemodul in einer Ultrafiltrationsunit gelöst, in dem der Produktstrom (=Feedstrom) und die Waschflüssigkeit (=Dialysat) strömend sind, wobei die Umpufferung ohne Kreislaufströmung des Produktstroms und der Waschflüssigkeit in das Hohlfaser-Ultrafiltrationsmodul erfolgt.

Der Produktstrom, der in die Kapillare befördert wird wie üblich in der Membrantechnik in der Anmeldung auch Feedstrom oder Feed genannt. Produktstrom, der aus den Kapillaren gefördert wird, wird wie üblich in der Membrantechnik in der Anmeldung auch Retentat genannt.

Üblicherweise werden kommerziell erhältliche Hohlfaser-Ultrafiltrationsmodule / Kapillar-Ultrafiltrationsmodul verwendet. Der Molecular Weight Cut-off (MWCO) der Ultrafiltrationsmembran ist dabei so zu wählen, dass die Membran das Zielprodukt derart in den Hohlfasern/Kapillaren zurückhält, dass ein Ausbeuteverlust ≤ 90 % bevorzugt ≤ 30 % und besonders bevorzugt ≤ 10 % nicht überschritten wird. Kleinere Moleküle und Ionen werden dabei nicht von der Membran zurückgehalten. Als Ausbeuteverlust ist der Verlust des durch die Membran permeierenden Produkts im Verhältnis zum Produkt in Feedstrom gemeint und wird üblicherweise durch Messung einer Probe mit einer der bekannten Methoden, wie z. B. chromatographische Methode, ELISA, usw. gemessen.

Von Vorteil ist, dass Kapillar-Ultrafiltrationsmodule dampfsterilisiert bzw. gammasterilisiert den Erfordernissen der pharmazeutischen Industrie entsprechend kommerziell erhältlich sind. Z. B. wurden in einer Testanlage Hämodialysemodule Revaclear 300 Capillary Dialyzer der Firma Gambro, umfassend Kapillare aus einem Polymerblend aus Polyaryethersulfone (PAES) und Polyvinylpyrrolidone (PVP) mit einem internen Durchmesser von 190 µm und mit einer Wanddicke von 35 µm verwendet (http://www.gambro.com/PageFiles/21256/Revaclear%20White%20Paper.pdf?epslanguage=en). Alternativ ist auch ein Hohlfasermodul wie z. B. die Process Scale Ultrafiltration Hollow Fiber Cartridge (UFP-750-E-55A) der Firma GE Healthcare anwendbar.

Erfindungsgemäß wird der Feedstrom kontrolliert in die Hohlfasern/Kapillaren des Ultrafiltrationsmoduls gefördert und eine Waschflüssigkeit kontrolliert in die Mantelseite gefördert. Das Retentat wird kontrolliert aus den Hohlfasern/Kapillaren herausgefördert und in die Anlage weitergeleitet. Überraschenderweise wurde festgestellt, dass die Umpufferung ohne Kreislaufströmung des Produktstroms erfolgreich durchgeführt werden kann. Dies erfolgt bereits bei der Nutzung eines einzigen Ultrafiltrationsmoduls. Es kann aber vorteilhaft sein mehrere Ultrafiltrationsmodule in Serie oder parallel zu verschalten. Bei dem Verfahren wird die Waschflüssigkeit entweder im Gleichstrom, im Kreuzstrom oder im Gegenstrom bevorzugt im Kreuzstrom, besonders bevorzugt im Gegenstrom zum Feedstrom gepumpt. Der Betrieb im Gegenstrom, in dem der Produktstrom (z. B. die Proteinlösung in Puffer A) dabei von unten nach oben durch die Hohlfasern/Kapillaren des Ultrafiltrationsmoduls strömt während die Waschflüssigkeit (Puffer S) mantelseitig üblicherweise von oben nach unten an den Hohlfasern/Kapillaren vorbeigeführt geführt wird, ermöglicht höhere Reinheit bei weniger Waschflüssigkeit. Ebenso kann der Produktstrom von oben nach unten durch die Kapillaren des Hämodialysemoduls strömen während die Waschflüssigkeit mantelseitig von unten nach oben an den Kapillaren vorbeigeführt wird. Überraschenderweise wurde festgestellt, dass diese einfache Lösung eine effiziente Umpufferung des Produktstroms auch im kontinuierlichen Betrieb ermöglicht.

Zur Beförderung des Produktstroms (=Feedstroms) und der Waschflüssigkeit wird jeweils eine Pumpe eingesetzt. Zur kontrollierten Abfuhr des Produktstroms aus den Kapillaren (=Retentats) wird üblicherweise eine weitere Pumpe oder ein Regelungsventil als Mittel zur geregelten Abfuhr des Produktstroms eingesetzt. Bevorzugt wird zur kontrollierten Abfuhr der Waschflüssigkeit das Druckverhältnis zwischen Kapillar- und Mantelseite (=Außenseite) eingestellt. Dies erfolgt üblicherweise ebenfalls durch Anwendung einer weiteren Pumpe oder eines Regelungsventils zur Abfuhr der Waschflüssigkeit als Mittel zur geregelten Abfuhr der Waschflüssigkeit.

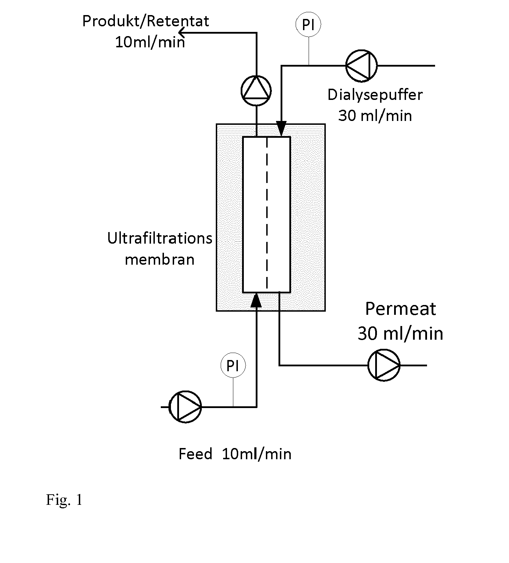

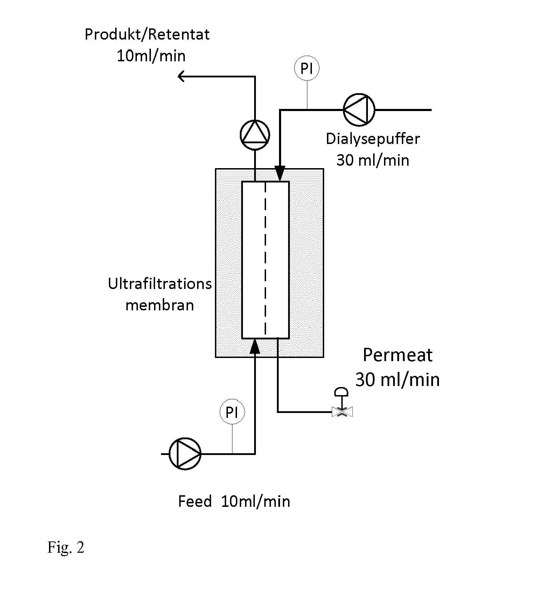

Erfindungsgemäße Ultrafiltrationsunits umfassend ein Kapillar-Ultrafiltrationsmodul sind in Fig. 1 und 2 exemplarisch dargestellt ohne sich darauf zu begrenzen.

Die Feedströmung und die Waschflüssigkeitströmung (=Dialyse Puffer) werden über Pumpen realisiert. Die Retentatströmung wird bevorzugt über eine zwangsfördernde Pumpe wie eine peristaltische Pumpe realisiert (Fig. 1), bzw. alternativ über ein Regelventil und eine Flussteuerung (Ausführungsform nicht dargestellt). Bevorzugt wird das Verhältnis der Drücke der Eingangsströme (Fig. 1, Feed und Dialysepuffer) geregelt um eine Kurzschlussströmung von einer Membranseite zur anderen zu vermeiden. Hierzu kann wie in Figur 1 dargestellt, eine zusätzliche vierte Pumpe im Permeatausgang eingesetzt werden (Fig. 1) oder bevorzugt der Permeatausgang durch ein Ventil oder hydrostatischen Druck (Fig. 2) angedrosselt werden.

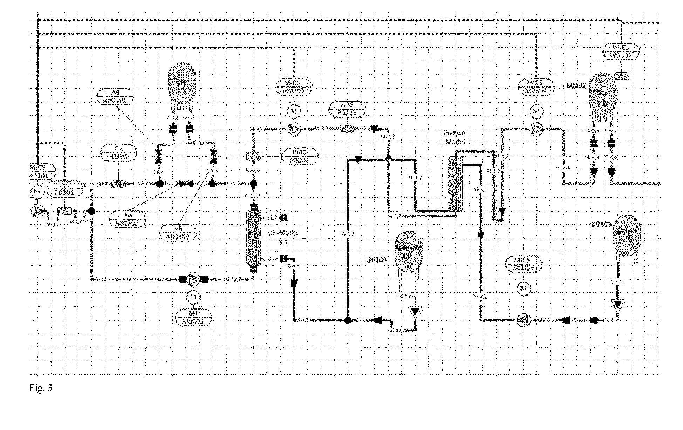

Eine weitere Ausführungsform der Ultrafiltrationsunit ist in Fig. 3 schematisch dargestellt ohne sich darauf zu begrenzen. Eine Pumpe M0303 fördert einen Volumenstrom in ein oder mehrere Hämodialysemodule (DF-Modul). Vorzugsweise werden ein oder mehrere Hämodialysemodule in Serie verbunden. Ein Drucksensor P0303 überwacht vorzugsweise den Feed-Druck der Ultrafiltrationsunit. Steigt dieser über den maximalen Druck, wird die Stufe abgeschaltet. Nach der Ultrafiltrationsunit pumpt die Pumpe M0304 bevorzugt genauso schnell wie M0303, um eine Konzentrationsveränderung des Produkts im Hämodialysemodul und eine Gelschichtbildung auf der Membran zu verhindern. Die Pumpe M0304 fördert das dialysierte Retentat in einen Zwischenbag B0302. Die Waschfüssigkeit wird mit der Pumpe M0305 in einer schnelleren Flussrate als M0303 in die Permeatseite der Hämodialysemodule (=Mantelseite der Kapillare) gepumpt. Die Flussrate der Pumpe M0303 wird dabei so hoch eingestellt, dass die gewünschten Konzentrationen an Puffer im Retentat erreicht werden. Die verbrauchte Waschflüssigkeit wird in Bag B0304 gesammelt.

Erster Gegenstand der vorliegenden Anmeldung ist eine Unit zur kontinuierlichen Ultrafiltration eines Produktstroms enthaltend biopharmazeutischen und biologischen makromolekularen Produkten umfassend mindestens ein Kapillar-Ultrafiltrationsmodul, wobei
- mindestens eine Pumpe, die den Produktstrom in die Kapillaren des Ultrafiltrationsmoduls fördert,
- ein Mittel zur geregelten Abfuhr des Produktstroms den Produktstrom aus den Kapillaren fördert und
- mindestens eine weitere Pumpe die Waschflüssigkeit über die Außenseite der Kapillaren strömt,
- ohne Maßnahmen zur Kreislaufströmung des Produktstroms und der Waschflüssigkeit in das Ultrafiltrationsmodul.

Bevorzugt wird ein Mittel zur geregelten Abfuhr der Waschflüssigkeit aus dem Ultrafiltrationsmodul eingesetzt.

Weiterer Gegenstand der Anmeldung ist ein Verfahren zur kontinuierlichen Ultrafiltration eines Feedstroms enthaltend biopharmazeutische und biologische makromolekulare Produkte in der erfindungsgemäßen Unit zur kontinuierlichen Ultrafiltration, wobei der Feedstrom mit einer Waschflüssigkeit über mindestens eine Kapillar-Ultrafiltrationsmembran eines Kapillar-Ultrafiltrationsmoduls gewaschen wird, dadurch dass der Feedstrom in das Kapillar gefördert wird und die Waschflüssigkeit über die Außenseite der Kapillare geströmt wird, wobei der Feedstrom in das Ultrafiltrationsmodul kontinuierlich zugeführt, und aus dem Ultrafiltrationsmodul kontinuierlich abgeführt wird, wobei der Feedstrom und die Waschflüssigkeit nicht im Kreislauf in das Ultrafiltrationsmodul gefördert wird.

Um einen effizienten Austausch kleiner Moleküle und Ionen zwischen den Membranseiten zu erreichen, wird möglichst der Nettovolumenstrom (= Volumenstrom im Mantelraum und im Kapillarenraum bleibt konstant) über die Membranen minimiert, so dass der Transport wie in einem Dialyseverfahren weitestgehend diffusiv erfolgt. Ein diffusiver Transport wird insbesondere erreicht, wenn die Verdünnung oder Aufkonzentrierung der nicht permeierenden Substanzen höchstens mit einem Faktor von 1 bis 5, bevorzugt mit einem Faktor von 1 bis 2 besonders bevorzugt mit einem Faktor 1 erfolgt.

Erfindungsgemäß können mehrere Ultrafiltrationsmodule in Serie oder parallel verschaltet werden. Die Anzahl der Ultrafiltrationsmodule in Serie ist dabei so auszuwählen, dass der gesamte Druckverlust über die Ultrafiltrationsmodule nicht 1 bar überschreitet. Die Anzahl der parallel geschalteten Ultrafiltrationsmodule ist so zu wählen, dass die Feedströmung in die Kapillaren nicht die maximale vom Hersteller empfohlene Flussrate übersteigt, bevorzugt ist eine Flussrate von 0,1 bis 15 % der empfohlenen Flussrate, besonders von 0,1 bis 5 % dieser Flussrate.

Z. B. wurden in der Testanlage Hämodialysemodule Revaclear 300 Capillary Dialyzer der Firma Gambro verwendet. In diesen Modulen sollte der Feedstrom 500 ml/min, bevorzugt 75 ml/min, noch besser 25 ml/min pro Modul nicht überschreiten. Module können so in Serie geschaltet werden, dass die gesamte Druckdifferenz über Eingang/Ausgang Module (=Druckverlust über die Membranen der Kapillar-Ultrafiltrationsmodule) die empfohlene Flussrate von 1 bar nicht überschreiten.

Um eine möglichst hohe Abreicherung der Verunreinigungen im Retentat zu erzielen, wird üblicherweise das Verhältnis der Strömung von Waschflüssigkeit (=Dialysatstrom) und Feedstrom möglichst hoch gewählt. Die exakte Höhe dieses Verhältnisses hängt von der geforderten Mindestabreicherung, wie auch der Diffusionsrate der Moleküle ab. Auch der Dialysatstrom in ein Modul sollte dabei den maximalen vom Hersteller empfohlenen Fluss nicht überschreiten. Auch aus wirtschaftlicher Sicht ist es sinnvoll den Dialysatstrom zu begrenzen. Es ist für einen batchweise betriebenen Diafiltrationsprozess gängige Praxis ein Verhältnis der Waschflüssigkeitsmenge zu Feedflüssigkeitsmenge von 3 - 6 einzustellen. In dem erfindungsgemäßen Verfahren kann eine vergleichbare Einstellung des Verhältnisses von Dialysatstroms zu Feedstroms von 3 - 6 als erster Ansatzpunkt gewählt werden und wird in der Regel experimentell an die Eigenschaften des Produkts angepasst.

Es zeigte sich überraschend, dass das Priming (Inbetriebnahme durch Waschen und Entlüften) der Dialysemodule entscheidend für die Waschgüte ist, da ansonsten nicht alle Kapillare ausgenutzt werden. Aufgrund der bevorzugt niedrigen Flussraten ist es eine besondere Schwierigkeit die Dialysemodule kapillarseitig wie auch mantelseitig zu entlüften.

Erfindungsgemäß wird bevorzugt jedes Dialysemodul vor Inbetriebnahme, sowohl die Feedseite, wie auch die Mantelseite mit einer Pufferlösung mit mindestens 10 % bis 100 %, bevorzugt mit 15 bis 100 % und besonders bevorzugt mit 30 bis 100 % der vom Hersteller empfohlenen Blutflussrate eingespült, bis keine Gasblasen mehr dem Dialysemodul entweichen. Es ist dabei kritisch, dass sowohl die Feedseite wie auch Mantelseite blasenfrei ist.

Erfindungsgemäß werden die Feed- und Dialysatströme so geregelt, dass diese Ströme eindeutig definiert sind, d. h. es zu keinen unerwünschten Nettoströmungen aus dem Feedraum (=Kapillarinnere) in den Dialysatraum (=Kapillaraußenseite/Mantelseite) oder umgekehrt kommen kann. Hierzu können folgende Maßnahmen getroffen werden:
- Überwachung des Produktstroms durch offline/online Signale UV, Leitfähigkeit, pH, Flussmessung, oder offline Messungen zur Bestimmung der Produktqualität, wie HPLC, Elisa usw.
- Es kann vorteilhaft sein, die Mantelseite (=Dialysatraum) oder Feedseite (=Feedraum) mit Druck zu beaufschlagen. Zu diesem Zweck, kann auf den jeweiligen Ausgängen ein Mittel zur geregelten Abfuhr der entsprechenden Flüssigkeit, entweder eine Pumpe oder ein Ventil, eingesetzt werden.

Die erfindungsgemäße Unit zur kontinuierlichen Ultrafiltration wird erfindungsgemäß in eine Produktionsanlage insbesondere in einer Produktionsanlage zur kontinuierlichen oder semikontinuierlichen Durchführung einer oder mehreren oben genannten Produktionsschritten integriert.

Weiterer Gegenstand der Anmeldung ist daher eine Produktionsanlage umfassend mindestens eine erfindungsgemäße Unit zur kontinuierlichen Ultrafiltration eines Produktstroms enthaltend biopharmazeutischen und biologischen makromolekularen Produkten.

Darüber hinaus finden in manchen der oben aufgeführten Produktionsschritten zur Herstellung von biopharmazeutischen und biologischen Produkten eine Sequenz von Verdünnungen und Konzentrationen des Produktstroms statt, die für eine kontinuierliche Verfahrensführung eine Herausforderung darstellen. Hiermit wird die erfindungsgemäße Produktionsanlage mit verschiedenen Durchflussraten betrieben, die vorzugsweise kompensiert werden sollten.

Vorzugsweise umfasst die erfindungsgemäße Produktionsanlage vor oder nach einer der Unit zur kontinuierlichen Ultrafiltration eines Produktstroms eine Unit zur Konzentration des Produktstroms (auch Konzentrationsunit genannt). Bevorzugt ist die erfindungsmäße Unit zur kontinuierlichen Ultrafiltration in der Produktionsanlage an der Austrittleitung einer Konzentrationsunit verbunden. Erfindungsgemäß umfasst die Konzentrationsunit wie in Fig. 3 exemplarisch dargestellt eine Rezirkulationsschleife (= Konzentrationsschleife), und in der Konzentrationsschleife ein oder mehrere Membranmodule mit einem Permeatausgang, eine Pumpe M0302 zur Umlaufströmung in der Konzentrationsschleife und ein Entlüftungsbag (keine Nummerierung). In der Konzentrationsschleife wird der Produktstrom kontinuierlich konzentriert. Eine Pumpe M0301 fördert den Produktstrom üblicherweise aus einem Vorlagebag in die Konzentrationsschleife. Als Membranmodul werden üblicherweise ein oder mehrere CFF-Ultrafiltrationsmodule (Cross Flow Filtration) alternativ ATF (Alternating Tangential Flow)-Ultrafiltrationsmodule, bevorzugt aber ein CFF-Modul verwendet. Z. B. wurden in der Testanlage CFF-Module RTP UFP-30-C-5S der Firma GE Healthcare LifeScience verwendet.

Die Förderrate von M0302 wird üblicherweise mindestens auf die minimale Überströmungsgeschwindigkeit eingestellt, die vom Membranhersteller angegeben wird. Z. B. wurden für das Modul RTP UFP-30-C-5S mindestens 21/min eingestellt.

Die Pumpe M303 fördert den Produktstrom aus der Konzentrationsschleife. Dabei wird die Förderrate der Pumpe M303 im Verhältnis zur Förderrate der Pumpe M301 eingestellt. Dieses Verhältnis entspricht dem gewünschten Aufkonzentrierungsfaktor. Dabei stellt sich am Drucksensor P0301 und P0302 ein Druck ein, der bewirkt, dass Flüssigkeit aus der Konzentrationsschleife durch die Membran permeiert, wobei jedoch das biologische Produkt weitestgehend zurückgehalten wird. Üblicherweise wird eine Membran verwendet, die mindestens 90 %, bevorzugt 90 - 95 %, besonders bevorzugt 95 - 100 % des biologischen Produkts zurückhält. In dem beschriebenen Aufbau wird eine zwangsfördernde Pumpe eingesetzt, die auch einen Vordruck halten kann, ohne dass die Flüssigkeit durch die Pumpe und nicht durch die Membran strömt. Vorzugsweise wird hier eine Schlauchpumpe eingesetzt. Als Alternative kann auch eine Regelventil-Flussmesser-Kombination eingesetzt werden, durch den der Permeatfluss eingestellt wird.

Das durch die Membran des Membranmoduls hindurchtretende Permeat wird durch den Permeatausgang im Behälter B0304 gesammelt. Der Drucksensor P0301 zwischen der Pumpe M0301 und der Konzentrationsschleife überwacht den Druck vor dem Membranmodul und kann zur Abschaltung von M0302 führen, falls ein maximaler Druck überschritten wird. In diesem Fall muss das Membranmodul ausgewechselt werden. Vorzugsweise wird die Konzentrationsschleife in diesem Fall als Ganzes durch eine neue sterilisierte ersetzt. Es ist aber durchaus bei der Nutzung von Membrankassetten üblich Kassetten parallel zu betreiben. Die Membrankassetten können seriell oder parallel, vorzugsweise parallel angeordnet sein. Üblicherweise misst ein Drucksensor P0302 den Druck hinter der Konzentrationsschleife, wodurch der Transmembrandruck bestimmt werden kann. Üblicherweise misst ein Durchflusssensor F0301 (nicht dargestellt) den Volumenstrom in der Konzentrationsschleife. In einem festen Verhältnis zu der Förderrate von M0301 fördert die Pumpe M0303 einen Volumenstrom aus der Konzentrationsschleife hinaus in die Ultrafiltrationseinheit.

Die Einhaltung der Keimfreiheit stellt eine weitere Herausforderung und Aufgabe für eine (semi-)kontinuierliche Produktionsanlage dar. In der erfindungsgemäßen Produktionsanlage werden alle Elemente durch Leitungen insb. Einweg-Leitungen miteinander verbunden. Beispielsweise werden biokompatible Leitungen Pharmed BPT® (temperaturbeständige Silikonschläuche) eingesetzt. Auch die weiteren Elemente der Anlage sind bevorzugt Einweg-Elemente; insbesondere werden Elemente selektiert aus der Gruppe von Einweg-Reaktoren, Einweg-Filtrationselemente, Einweg-Ventile, Einweg-Sensoren (Fluss, pH, Leitfähigkeit, UV, Druck), Einweg-Zellrückhaltesysteme, Einweg-Schläuche, Einweg-Membranen, Einweg-Verbindungsstücke, Einweg-Chromatographiesäulen, Einweg-Behälter, Einweg Probennahmesysteme verwendet. Auch Mittel zur Beförderung von Flüssigkeit insbesondere Pumpen sind vorzugsweise Einweg-Pumpen.

Für den Wechsel des Membranmoduls und / oder eines Ultrafiltrationsmoduls wird üblicherweise der Produktstrom unterbrochen und ein Zwischenbag (Vorlagebag B0301 in Fig. 3) füllt sich auf für die Dauer des Wechsels.

Durch die Anwendung von Units zur Sterilfiltration zur weiteren Reduzierung des BioBurdens kann die Lebensdauer der jeweiligen Module so verbessert werden, dass eine Unterbrechung und der Fang des Produktstroms in einem Zwischenbag für die Dauer des Wechsels vertretbar ist.

Alternativ zur Konzentrationsschleife kann als Unit zur Konzentration des Produkstroms eine oder mehrere Cadence™ Single-Pass Tangential Flow Filtrationskassetten der Firma Pall Corp verwendet werden (US 7.682.511 B2, http://www.pall.com/main/biopharmaceuticals/product.page?id=52742).

Durch den Konzentrierungsschritt wird der Volumenstrom deutlich verringert und die Produktkonzentration erhöht.

In einer besonderen Ausführungsform umfasst die erfindungsgemäße Produktionsanlage eine oder mehrere Units zur Chromatographie angeschlossen nach der Unit zur kontinuierlichen Ultrafiltration des Produktstroms.

### Beispiel und Testanlage:

Zur Untersuchung der Anwendbarkeit der erfindungsgemäßen Lösung zur Umpufferung eines Produktstroms aus einer Fermentationsbrühe wurde eine Testanlage gemäß Fig. 3 exemplarisch aufgebaut. Als Leitungen der Testanlage wurden Pharmed BPT® Leitungen mit einem Innendurchmesser von 3,2 mm verwendet. Über die Elemente der Fig. 3 hinaus umfasste die Testanlage ein 10 L Behälter gefüllt mit einer von Zellen befreiten Fermentationsbrühe als Testproduktstrom. Eine erste Pumpe (M0301) verbunden mit Hilfe von Leitungen mit dem Behälter (nicht dargestellt) förderte den Produktstrom wie auf Fig. 3 dargestellt vorerst in die Konzentrationsunit. Als Testmolekül wurde ein Immunoglobulin G-Antikörper (IgG-Antikörper) ausgewählt.

Die Konzentrationsunit umfasste eine Pumpe M0302 zur Umlaufströmung in die Konzentrationsschleife, ein Entlüftungsbag (keine Nummerierung) verbunden mit Hilfe von Leitungen und manuellen Quetschventilen AB0301, AB0302 und AB0303 an die Konzentrationsschleife und ein Membranmodul (UF-Modul 3.1). Als Membranmodul wurde exemplarisch ein CFF-Ultrafiltrationsmodul RTP UFP-30-C-5S der Firma GE Healthcare LifeScience verwendet.

Die Förderrate von M0302 wurde auf die minimale Überströmungsgeschwindigkeit eingestellt, die vom Membranhersteller angegeben wird. Z. B. wurde für das Modul RTP UFP-30-C-5S mindestens 2 l/min eingestellt. Vor der eigentlichen Aufkonzentrierung wird zunächst die Konzentrationsschleife entlüftet in dem AB0301 und AB0303 und AB0302 teilweise geöffnet werden. Wenn die Konzentrierungsschleife blasenfrei ist, wird AB0302 vollständig geöffnet und AB0301 und AB0302 geschlossen.

Die Pumpe M0303 förderte den Produktstrom aus der Konzentrationsschleife. Dabei wurde die Förderrate der Pumpe M0303 im Verhältnis zur Förderrate der Pumpe M0301 eingestellt, der dem gewünschten Aufkonzentrierungsfaktor entsprach. Am Drucksensor P0301 und P0302 stellte sich ein Druck ein, der bewirkte, dass Flüssigkeit aus der Konzentrationsschleife durch die Membran permeierte, wobei jedoch das biologische Produkt weitestgehend zurückgehalten wurde.

Das durch die Membran des Membranmoduls hindurchtretende Permeat wurde im Behälter B0304 gesammelt. Der Drucksensor P0301 zwischen der Pumpe M0301 und der Konzentrationsschleife überwachte den Druck vor dem Membranmodul und konnte zur Abschaltung von M0302 führen, falls ein maximaler Druck überschritten wurde. In diesem Fall müsste das Membranmodul ausgewechselt werden. Der Drucksensor P0302 maß den Druck hinter der Konzentrationsschleife, wodurch der Transmembrandruck bestimmt werden konnte. Ein Durchflusssensor F0301 (nicht dargestellt) maß den Volumenstrom in der Konzentrationsschleife.

In einem festen Verhältnis zu der Förderrate von M0301 förderte die Pumpe M0303 einen Volumenstrom aus der Konzentrationseinheit hinaus in die folgende Ultrafiltrationsunit.

In der Testanlage umfasste die Ultrafiltrationsunit, wie in Fig. 3 dargestellt ein Hämodialysemodul (Dialyse Modul) Revaclear 300 Capillary Dialyzer der Firma Gambro umfassend Kapillare aus einem Polymerblend aus Polyaryethersulfone (PAES) und Polyvinylpyrrolidone (PVP) mit einem internen Durchmesser von 190 µm und mit einer Wanddicke von 35 µm verwendet (http://www.gambro.com/PageFiles/21256/Revaclear%20White%20Paper.pdf?epslanguage=en). Pumpe M0303 förderte einen Volumenstrom in das Hämodialysemodul. Ein Drucksensor P0303 überwachte den Feed-Druck der Ultrafiltrationsunit.

Vor Inbetriebnahme des Hämodialysemoduls wurden sowohl die Feedseite wie auch die Mantelseite mit einer Pufferlösung (=Waschflüssigkeit) mit 80 - 100 ml/min Blutflussrate gewaschen, bis keine Gasblasen mehr dem Modul entwichen (Priming). Es war dabei kritisch, dass sowohl die Feedseite wie auch Mantelseite blasenfrei waren.

Für die Dialyse des Produktstroms wurde dann der Feedstrom auf 3 ml/min pro Modul eingestellt.

Der Dialysepuffer wurde mit der Pumpe M0305 in einer Flussrate von 3 bzw. 6 ml/min als der von M0303 im Gegenstrom zum Feedstrom in die Permeatseite der Hämodialysemodule gepumpt. Die Flussrate in M0303 wurde dabei so hoch eingestellt, dass die gewünschten Konzentrationen an Puffern im Produkt erreicht wurden. Die verbrauchte Waschflüssigkeit wurde in Bag B0304 gesammelt.

Die Feed- und Dialysatströme wurden so geregelt, dass diese Ströme eindeutig definiert sind, d. h. es zu keinen unerwünschten Nettoströmungen aus dem Feedraum (Kapillarinnnere) in den Dialysatraum (Kapillaraußenseite) oder umgekehrt kommen kann.

Hierzu wurden folgende Maßnahmen getroffen:
Überwachung des Produktstroms durch offline/online Signale UV, Leitfähigkeit, pH, Flussmessung, oder offline Messungen zur Bestimmung der Produktqualität, wie HPLC, Elisa.

Nach der Ultrafiltrationsunit förderte die Pumpe M0304 genauso schnell wie M0303, um eine Konzentrationsveränderung des Produkts im Dialysemodul und eine Gelschichtbildung auf der Membran zu verhindern, den konzentrierten und dialysierten Produktstrom in einen Zwischenbag B0302, dessen Füllung mit einer Wägezelle WO302 überwacht wurde.

Ausbeuteverlust und erfolgreiche Umpufferung des Produktstroms wurden durch die Konzentrationsbestimmungen von Produkt, Glukose und Salz geprüft und ergaben:
In einem ersten Experiment wurde der Ionenaustausch mit einem Produktstrom ohne biologisches Produkt getestet.

Tabelle A zeigt die Messungen der Leitfähigkeit des Feeds (LF Feed) und des Retentats (LF Retentat) bei verschiedenen Sollflussraten der jeweiligen Pumpen.

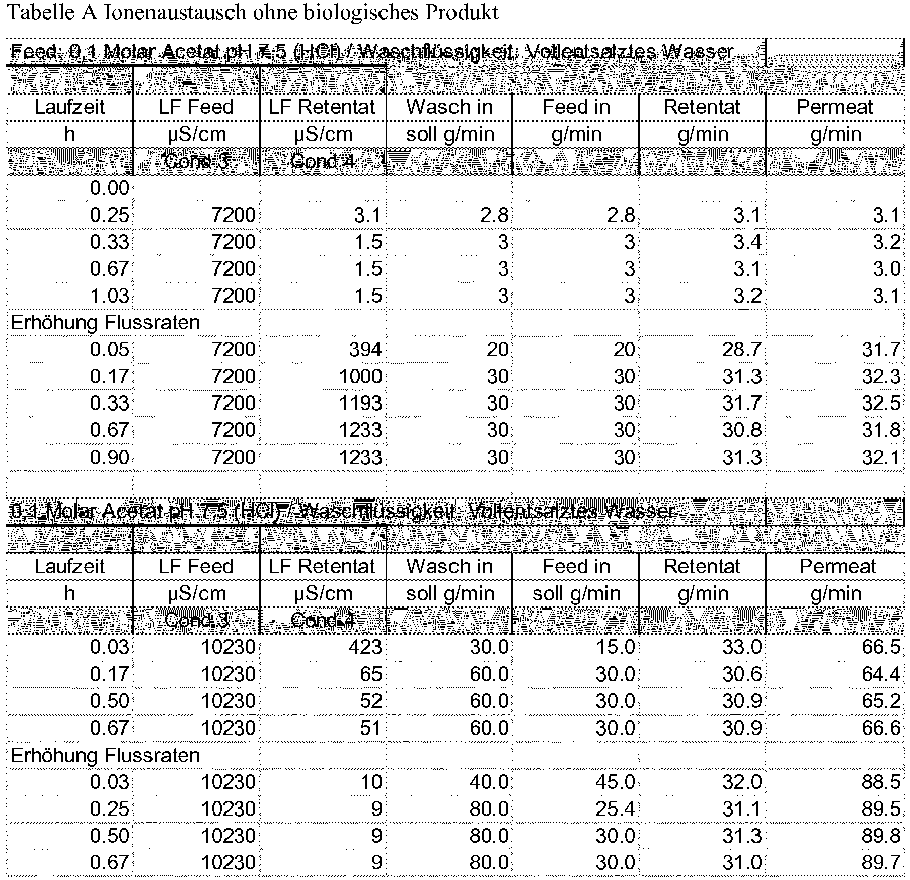

In einem weiteren Versuch wurde der Ionenaustausch mit einem Produktstrom mit IgG-Antikörper (gemessen über HPLC Prot A) getestet.

Tabelle B zeigt die Messungen der Leitfähigkeit des Retentats (LF Retentat) bei verschiedenen Sollflussraten der jeweiligen Pumpen und die Konzentration des IgG Antikörper im Retentat gemessen mittels HPLC ProtA.

Auch im Permeat wurde die Konzentration des IgGin der Sammelprobe bei erhöhter Flussrate mit der o. g. Methode ermittelt, zur Berechnung des Ausbeuteverlusts. Der Ausbeuteverlust im Permeat betrug demnach 1.4 %.

Die Messung der Konzentration an Natrium Acetat mittels Ionenchromatographie zeigt die erfolgreiche Umpufferung des Produktstroms.

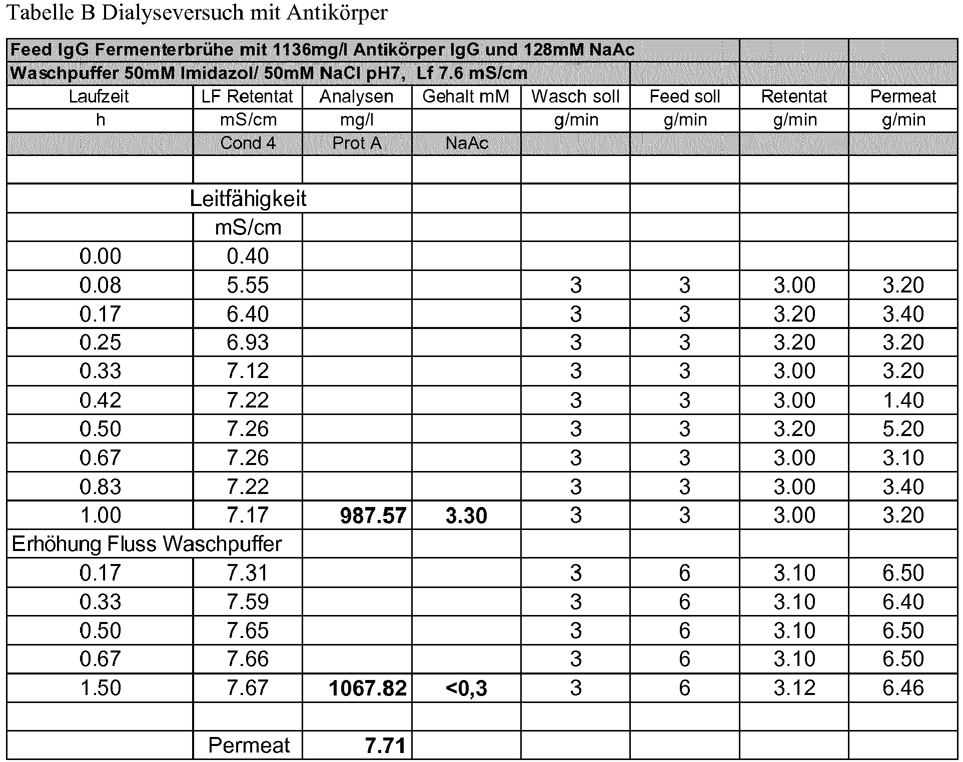

Die Arbeiten, die zu dieser Anmeldung geführt haben, wurden gemäß der **Finanzhilfevereinbarung "Bio.NRW: MoBiDiK -** Modulare Bioproduktion - Disposable und Kontinuierlich" im Rahmen des Europäischen Fonds für regionale Entwicklung (EFRE) gefördert.

## Patentansprüche

1. Unit zur kontinuierlichen Ultrafiltration eines Produktstroms enthaltend biopharmazeutische und biologische makromolekulare Produkte umfassend mindestens ein Kapillar-Ultrafiltrationsmodul, wobei
- mindestens eine Pumpe den Produktstrom in die Kapillaren des Ultrafiltrationsmoduls fördert,
- ein Mittel zur geregelten Abfuhr des Produktstroms den Produktstrom aus den Kapillaren fördert und
- mindestens eine weitere Pumpe die Waschflüssigkeit über die Außenseite der Kapillaren strömt,
- die Unit keine Maßnahme zur Kreislaufströmung des Produktstroms und der Waschflüssigkeit in das Ultrafiltrationsmodul umfasst.

2. Unit nach Anspruch 1, umfassend ein Mittel zur geregelten Abfuhr der Waschflüssigkeit aus dem Ultrafiltrationsmodul eingesetzt.

3. Unit nach einem der Ansprüche 1 oder 2, wobei mehrere Kapillar-Ultrafiltrationsmodule in Serie oder in Parallel verschaltet sind.

4. Verfahren zur kontinuierlichen Ultrafiltration eines Feedstroms enthaltend biopharmazeutische und biologische makromolekulare Produkte in der Unit zur kontinuierlichen Ultrafiltration, wobei der Feedstrom mit einer Waschflüssigkeit über mindestens eine Kapillar-Ultrafiltrationsmembran eines Kapillar-Ultrafiltrationsmoduls gewaschen wird, dadurch dass der Feedstrom in das Kapillar gefördert wird und die Waschflüssigkeit über die Außenseite der Kapillare geströmt wird, wobei der Feedstrom in das Ultrafiltrationsmodul kontinuierlich zugeführt, und aus dem Ultrafiltrationsmodul kontinuierlich abgeführt wird, wobei der Feedstrom und die Waschflüssigkeit nicht im Kreislauf in das Ultrafiltrationsmodul gefördert wird.

5. Verfahren nach Anspruch 4, wobei in der Unit zur kontinuierlichen Ultrafiltration das Produkt im Feedstrom maximal um ein Faktor 2 konzentriert bzw. um ein Faktor 2 verdünnt wird.

6. Verfahren nach einem der Ansprüche 4 oder 5, wobei die Waschflüssigkeit im Kreuzstrom oder Gegenstrom, bevorzugt im Gegenstrom zum Feedstrom betrieben wird.

7. Verfahren nach einem der Ansprüche 4 bis 6, wobei mehrere Kapillar-Ultrafiltrationsmodule in Serie oder in Parallel und der maximale Druckverlust über die Membranen der Kapillar-Ultrafiltrationsmodule 1 bar nicht überschreitet.

8. Produktionsanlage umfassend mindestens eine Unit zur kontinuierlichen Ultrafiltration eines Produktstroms enthaltend biopharmazeutische und biologische makromolekulare Produkte gemäß einem der Ansprüche 1 bis 3.

9. Produktionsanlage nach Anspruch 8, weiterhin umfassend mindestens eine Konzentrationsunit verbunden mit der Unit zur kontinuierlichen Ultrafiltration.

10. Produktionsanlage nach Anspruch 9, wobei die Konzentrationsunit umfasst:
- eine Pumpe zur Beförderung des Produktstroms in eine Konzentrationsschleife umfassend ein oder mehrere Membranmodule mit einem Permeatausgang, eine Pumpe zur Umlaufströmung in der Konzentrationsschleife und ein Entlüftungsbag, sowie
- eine Pumpe zur Beförderung der proteinhaltigen Lösung aus der Konzentrationsschleife.
